# EUROPÄISCHE PATENTSCHRIFT

(11) **EP 0 685 492 B1**
(45) Veröffentlichungstag und Bekanntmachung des Hinweises auf die Patenterteilung: **21.11.2001**
(21) Anmeldenummer: 95107280.0
(22) Anmeldetag: 13.05.1995
(51) Int. Cl.: C07K 14/805, C07K 1/34

(54) **Verfahren zur Herstellung molekulareinheitlicher hyperpolymerer Hämoglobine**
Process for the preparation of hyperpolymers of haemoglobin with uniform molecular weight
Procédé de préparation d'hyperpolymères d'hémoglobine de poids moléculaire uniforme

(30) Priorität: 31.05.1994 DE 4418973
(43) Veröffentlichungstag der Anmeldung: 06.12.1995
(60) Teilanmeldung: 01101484.2
(73) Patentinhaber: SanguiBioTech AG, 58455 Wittenan der Ruhr (DE)
(72) Erfinder: Barnikol, Wolfgang, Prof. Dr. Dr., 55128 Mainz (DE)
(74) Vertreter: Beil, Hans Christoph, Dr.

(56) Entgegenhaltungen:
- EP-A- 0 078 961
- EP-A- 0 201 618
- WO-A-87/07832
- WO-A-88/03408
- WO-A-90/13309
- WO-A-92/09630
- WO-A-92/21702
- DE-A- 2 714 252
- DE-A- 3 714 351
- DE-A- 3 841 105
- FR-A- 2 225 171
- PÖTZSCHKE, H. ET AL.: "Vernetzte globuläre Proteine", MACROMOL.CHEM.PHYS., , 1996, Band 197, Nr. , Seiten 1419 - 1437

## Beschreibung

Die Erfindung betrifft ein Verfahren zur Herstellung molekular - einheitlicher hyperpolymerer Hämoglobine durch Ultrafiltration.

Chemische Modifikationen von Hämoglobinen, z.B. Veränderungen der Sauerstoff-Affinität oder Polymerisation des Moleküls, werden vorgenommen, um sogenannte Blutersatzmedien bereitzustellen, die beim Menschen die Sauerstoff - Transport-Funktion unterstützen können.

Diese Blutersatzmedien können beispielsweise bei einem Verkehrsunfall mit schwer zu beherrschenden Blutungen, bei Unfällen unter Blutverlust oder für den Fall eines Infektionsrisikos (Hepatitis, AIDS) als Ersatz für eine momentan nicht verfügbare, passende Blutkonserve infundiert werden; dies gilt auch dann, wenn ein Mensch, z.B. in den genannten Fällen in einen Volumen - Mangel - Schock geraten ist. Es ist möglich, daß eine sauerstoffübertragende Blutersatzlösung einen Volumen - Mangel
- Schock eher durchbrechen kann, als ein eine Blutkonserve, da die Erythrozyten bekanntlich in der Konserve versteift sind und dadurch eine verringerte Kapillardurchgängigkeit aufweisen.
Ein sauerstofftransportierender Blutersatz ist gegenüber einer Blutkonserve auch für den Fall günstiger, wenn die Gefahr einer immunologischen Überreaktion besteht. An Tierversuchen ist gezeigt worden, das mit sauerstoffübertragenden Blutersatzlösungen ein Volumen - Mangel - Schock wirksamer bekämpft werden kann, als mit einfachen Plasmaexpandern (Übersichtsartikel hierzu: R. Pabst, Med.Klein. 72 (1977), 1555 - 1562).
Es ist ferner zu erwarten, daß auch chronische Durchblutungsstörungen (beispielsweise koronare, cerebrale und periphere) mit Hilfe geeigneter Polyhämoglobinlösungen wirksam bekämpft werden können. Nicht zuletzt lassen sich Sauerstoff - Mangelzustände ohne Durchblutungsverhinderung z.B.: chronische Anämien, mit solchen Lösungen bekämpfen. Diese Indikation besitzt schätzungsweise sogar einen zehnfach größeren Anwendungsbereich.

Zur Herstellung sauerstoffübertragender Blutersatzmedien sind bereits verschiedene Wege beschritten worden, nämlich:
1. Verwendung von Emulsionen von Fluorkohlenwasserstoffen, in welchen der Sauerstoff sehr gut löslich ist (Übersichtsartikel hierzu: Hirlinger et al., Anästhesist 31 (1982), 660-666). Diese Methode hat jedoch den Nachteil, daß bei Anwendung von Fluorkohlenwasserstoffen gewebliche Reaktionen auftreten.
2. Die Mikroverkapselung konzentrierter Hämoglobinlösungen in Phospholipid-Vesikeln zu sogenannten "künstlichen Erythrozyten" (Übersichtsartikel hierzu: Gaber et al.,Encapsulation of hemoglobin in Phospholipid vesicles; Preparation and Properties of a Red Cell Surrogate in "The red Cell Sixth Ann Arbor Conference", G.J. Brewer (Herausgeber), Alan R. Liss, Inc. New York, (1984), 179-190). Diese Methode befindet sich derzeit noch im Entwicklungsstadium der Tierversuche. Außerdem besteht hier die Gefahr einer Lipoid - Überlastung des Organismus durch vesikelbildende Lipoide.
3. Herstellung geeigneter Hämoglobinlösungen. Diese Methode bietet die besten Erfolgsaussichten.

Die DE - OS 2417619 beschreibt beispielsweise polymerisiertes, verknüpftes Hämoglobin als Plasmaprotein - Ersatz, wobei Dicarboxilidat - verknüpftes Hämoglobin hergestellt wird.

Die DE - OS 2714252 beschreibt Pyridoxalphosphat - verknüpftes Hämoglobin.

Die DE - OS 3029307 betrifft ein Blutersatzmittel, daß durch kovalente Verknüpfung von Polysaccharid, beispielsweise Dextran, mit zellfreiem Hämoglobin hergestellt wird.

Die BE - PS 838933 beschreibt die Herstellung eines wasserlöslichen, verknüpften, polymerisierten Hämoglobins durch Umsetzung freien Hämoglobins mit einem polyfunktionellen verknüpfenden Agens und anschließendem Abstoppen der Reaktion mit einem inaktivierendem Mittel. Es wird ein polymeres Hämoglobin mit einem Molekulargewicht von 64 000 bis 1 000 000 Dalton erhalten.

Die US - PS 4001401 betrifft ein verknüpftes, polymerisiertes Hämoglobin, als Blutersatz und Plasmaexpander mit einem Molekulargewicht von 64 000 bis 1 000 000 Dalton, das mittels der verknüpfenden Agenzien Glutaraldehyd, Hexamethylendiisocyanat oder Butadiendiepoxid gewonnen wird.

Die EP - PS 0201618 betrifft ein Verfahren, aus hoch konzentrierten Lösungen von monomerem Hämoglobin extrem hochmolekulare, kompakte, lösliche Polymere, sogenannte Hyperpolymere, des Hämoglobins herzustellen.

In der DE - PS 3714351 ist dieses Verfahren in sofern vereinfacht, als direkt Erythrozyten Verwendung finden können und das Vernetzungsmittel nicht mehr in einer Lipid - Phase zugesetzt werden muß.

Bei der Präparation geeigneter modifizierter Hämoglobinlösungen für eine routinemäßige, klinisch - praktische Nutzung tritt unteranderem die Notwendigkeit auf, die Viskosität der Lösung möglichst klein zu halten. Die Viskosität des Blutes bestimmt entscheidend den sogenannten totalen peripheren Widerstand des Organismus mit; jener darf keinesfalls zu groß sein, weil das der Kreislauf nicht toleriert.
Im Fall von Polymerlösungen und das gilt speziell auch für Hämoglobinpolymere, treten solche Probleme vor allem dann auf, wenn eine Polymerisation zu Kettenmolekülen erfolgt. Damit die Viskosität der Lösungen klein bleibt, sollte das Polymer möglichst kompakt sein und kein durchspültes Fadenmolekül, um bei möglichst geringer Viskosität des Plasmas, eine möglichst große Konzentration des Sauerstoffträgers applizieren zu können.
Das Einstein'sche Viskositätsgesetz besagt, daß einheitlich große Kugeln in einer Flüssigkeit, und zwar unabhängig von ihrem Radius, eine minimale Viskosität aufweisen.
Daher wäre es zur Verringerung der Viskosität extrem wichtig, möglichst einheitliche sauerstofftragende Moleküle, wie sie im übrigen auch in der Natur (Regenwurm) zu finden sind, herstellen können. Dann könnte das Molekulargewicht des Sauerstoffträgers sehr hoch gemacht werden, damit andererseits die Forderung nach einem vernachlässigbaren kolloidosmotischen Druck erfüllt ist.

Für den Fall eines sogenannten hypokotischen Blutzusatzes auf der Basis von Hämoglobinlösungen besteht außerdem die Notwendigkeit, speziell niedermolekulare Anteile der Hyperpolymeren unbedingt zu entfernen.

Mit der EP - PS 0 201 618 und der DE - PS 37 14 351 kann das Problem der Verknüpfung von Hämoglobin zu kompakten aber löslichen Riesenmolekülen als gelöst angesehen werden. Die dort beschriebenen Verfahren führen jedoch zu einem Hyperpolymergemisch mit einer breiten Verteilung des Molekulargewichtes und einer stark überproportionalen Zunahme der Viskosität bei steigender Konzentration (Barnikol and Burkhard, Adv. Exp. Biol. Med. 248 (1989) 335-340).

Die DE OS 3841 105 betrifft ein Verfahren zur Aufreinigung und Modifikation von polymerisiertem Hämoglobin, wobei durch fraktioniertes Lösen Polymere verschiedener Molkulargewichte abgetrennt erhalten werden können.
DEA 3714 351 offenbart eine Methode zur insbesondere Polymerisation von Hämoglobin durch invitro Reaktion mit intakten Erythrozyten. Eine Abtrennung von schädlichen Substanzen kann z.B. durch Abfiltrieren erfolgen. WO 87/07832 beschreibt die Herstellung eines Blutersatzstoffes und Reinigung durch (Ultra)filtration. Eine Auftrennung in unterschiedliche Molekulargewichtsbereiche ist hierbei nicht angegeben. WO 92109630 betrifft die Herstellung eines Blutersatzstoffes mit einem Molekulargewicht von wenigstens 80 % von 64 000 D. FR-A 2 225 171 beschreibt Hämoglobin mit einem Molekulargewicht von 68 000 und 85 000 oder 250 000. Mittels (NH4)2 S04 in einer Anwendungskonzentration bis zu 25 % Sättigung können nieder molekulare Anteile entfernt werden.
EP-A 00 78 961 betrifft die Herstellung von vernetztem Hämoglobin, wobei mittels Ultrafiltration im Kreislauf mit einem Filter von 1 00 000 D monomeres Hämoglobin abgetrennt wird.

Aufgabe der vorliegenden Erfindung ist es deshalb, ein Verfahren zu schaffen, mit dem aus einer bekannten Hämoglobin-Hyperpolymer-Lösung mit Hyperpolymeren unterschiedlicher Molelkulargewichte molekular-einheitliche Hämoglobin-Hyperpolymere abgetrennt werden können.

Die Lösung dieser Aufgabe besteht darin, eine an sich bekannte Lösung hypolpolymerer Hämoglobine unterschiedlichen Molekulargewichtes einem Ultrafiltrationsprozeß zu unterwerfen, wobei ein Filter mit einer normalen Trenngrenze von 10 ⁶ g/mol verwendet wird. Das erhaltene hyperpolymere Hämoglobin weist einen Endmolekulargewichtsbereich von etwa 500 000- 15 x 10 ⁶ g/mol auf.

Die genannte Methode ist im Stand der Technik an sich, als Verfahren zur molekular - gewichtsabhängigen Abtrennung von solchen Proteinen bekannt, deren Größe bzw. Gewicht maximal durch eine Quartärstruktur bestimmt ist, wobei jedoch ein Filter mit einer geringeren Trenngrenze eingesetzt wird. Der obere Molekulargewichtsbereich liegt nach bisherigen Erfahrungen bei etwa 500 000. Dies ist auch die obere Grenze der im Handel erhältlichen Markerproteinen zur vergleichenden Molekulargewichtsbestimmungen. Bei besonders großen Proteinen besteht bekanntermaßen das Problem, daß diese oft nicht als Ganzes abgetrennt werden, sondern aus bisher größtenteils noch unbekannten Gründen ganz oder teilweise zerfallen und man infolgedessen nur Untereinheiten abgetrennt erhält.

Bei den der Erfindung zugrundeliegenden Hämoglobin - Hyperpolymeren handelt es sich im Unterschied zu den gewöhnlich vorliegenden Proteinen um Riesenmoleküle, die erst in jüngster Zeit erstmals synthetisiert wurden und deren Größe bzw. Gewicht je nach dem Polymerisationsgrad, ein Hundert - bis Mehrhundertfaches, des an sich schon relativ großen und schweren, quartärstrukturierten Hämoglobin - Moleküls beträgt.

Es wurde nun überraschenderweise gefunden, daß mit dem an sich bekannten Verfahren der Ultrafiltration auch aus einem Gemisch solcher hyperpolymeren Hämoglobin - Riesenmoleküle, Fraktionen mit einheitlichem Molekulargewicht abgetrennt werden können. Bei diesen Hämoglobin - Riesenmolekülen handelt es sich nach ersten und vorläufigen polymeranalytischen Studien zur Viskosität, Lichtstreuung und Ultrazentrifugation (Pötschke, Barnikol, Biol. Chem. Hoppe-Seyler, 373 (1992) 811; und Massenkeil, Kirste,Pötschke, Barnikol, Biol. Chem. Hoppe - Seyler, 373 (1992) 798) um Kettenmoleküle.

Hinsichtlich einer Ultrafiltration war bei solchen Riesenmolekülen aufgrund des allgemeinen Kenntnisstandes viel eher zu erwarten gewesen, daß eine Passagierung durch die im wesentlichen kreisförmigen Poren eines Ultrafilters schon aufgrund des Kettencharakters der Hämoglobin - Hyperpolymere zu keiner molekulargewichtsspezifischen Abtrennung führt. Dafür sprach auch, daß es bisher nicht gelang, die hochmolekularen Anteile im Filtrat zu entfernen. Entgegen jener Erwartung wurde jedoch überraschenderweise gefunden, daß es mit Hilfe der Ultrafiltration möglich ist, aus einer Rohpolymerisat mit breitem Molekulargewichtsbereich die niedermolekularen Anteile im Retentat zu entfernen. Damit geht als weiterer Vorteil einher, daß der kolloidosmotische Druck des Polymerisates entscheidend gesenkt wird.

Das an sich bekannte Verfahren, durch zügiges Waschen einen frisch vernetzten und zunächst noch unlöslichen Hämoglobin - Hyperpolymerisats überflüssigen Reaktanten sowie monomere und ogliomere Hämoglobin - Molekülen zu entfernen, kann mit dem vorgenannten Verfahren problemlos kombiniert werden. Es können somit auf unerwartet einfache Art und Weise Hämoglobin - Hyperpolymerisate gewonnen werden, deren Bestandteile hinsichtlich ihres Molekulargewichts ein Höchstmaß an Einheitlichkeit aufweisen.

Somit ist es möglich den genannten Prozeß auf die Art (unterschiedliche Vernetzer und/oder unterschiedliche Hämoglobine, z.B. Rinder -, Schweine -, Menschenhämoglobin) des jeweils vorliegenden und zu trennenden Hämoglobin-Hyperpolymer - Gemisches anzuwenden und auf diese Weise eine vorteilhaft hohe Trennwirkung für Hämoglobine mit einem Molekulargewicht innerhalb sehr enger Bereichsgrenzen zu erzielen.
Die Erfindung wird anhand des folgenden Ausführungsbeispieles näher erläutert.

### Beispiel Ultrafiltration

Menschliches Hämoglobin wurde gemäß bekannter Vorschrift (Pötzschke, Barnikol, Biomater, Art Cells and Immob. Biotechn. 20 (1992) 287 - 291) mit Hilfe von Glutardialdehyd zu Hyperpolymeren vernetzt. Eine etwa 20%ige Lösung des Hyperpolymeren wurde sodann in der Elektrolytlösung BIKU (in mmol/l: NaCI; KCI 4,5; NahCO₃ 20;0,2 g/l NaN₃) einer Ultrafiltration unterzogen. Dabei fand ein Filter mit einer normalen Trenngrenze von 10⁶ g/mol Verwendung. Der Filtrationsprozeß erfolgte mit dem ungefähr zwanzigfachen Flüssigkeitsvolumen der ursprünglichen Lösung.
Mit Sephacryl S - 400 HR (Deutsche Pharmacia, Freiburg/D) lassen sich die hyperpolymeren Hämoglobine bezüglich ihrer Polymolarität analysieren. Die Grenz-Molekulargewichte des unfiltrierten Produktes waren 65 000 (unten)
und 15 x 10⁶ g/mol (oben), nach der Ultrafiltration betrugen die des Retentates 500 000 bzw. 15 x 10⁶ g/mol.

Mit Hilfe der Ultrafiltration ist es somit gelungen, aus dem Rohpolymerisat, dessen Bestandteile einen sehr breiten Molekulargewichtsbereich abdecken, die niedermolekularen Anteile zu entfernen und damit den kolloidosmotischen Druck des Polymerisates entscheidend zu senken.

## Patentansprüche

1. Verfahren zur Herstellung molekular- einheitlicher hyperpolymerer Hämoglobine, **dadurch gekennzeichnet, daß** eine an sich bekannte Lösung hyperpolymerer Hämoglobine unterschiedlichen Molekulargewichts mit einem Ausgangsmolekulargewicht von 65 000 bis 15 x 10⁶ g/mol dem folgenden Prozess unterworfen wird:
einem Ultrafiltrationsprozess, unter Verwendung eines Filters, mit einer normalen Trenngrenze von 10⁶ g/mol, wobei hyperpolymere Hämoglobine mit einem Endmolekulargewichtsbereich von etwa 500 000 - 15 10⁶ g/mol erhalten werden.

## Claims

1. Process for the preparation of haemoglobin hyperpolymers of uniform molecular weight, **characterized in that** a solution, known per se, of haemoglobin hyperpolymers of different molecular weight, with an initial molecular weight of 65,000 to 15 x 10⁶ g/mol, is subjected to an ultrafiltration process using a filter with a normal cut-off threshold of 10⁶ g/mol to give haemoglobin. hyperpolymers with a final molecular weight ranging from about 500,000 to 15 x 10⁶ g/mol.

## Revendications

1. Procédé de préparation d'hémoglobines hyperpolymères de masse molaire uniforme, **caractérisé en ce que** l'on soumet une solution connue en soi d'hémoglobines hyperpolymères de masse molaire variable ayant une masse molaire de départ de 65 000 à 15 x 10⁶ g/mol à l'opération suivante: une opération d'ulltrafiltration à l'aide d'un filtre ayant un seuil de coupure normal de 10⁶ g/mol, grâce à quoi on obtient des hémoglobines hyperpolymères ayant une masse molaire finale comprise entre environ 500 000 et 15 x 10⁶ g/mol.
